# EUROPEAN PATENT APPLICATION

(11) **EP 2 871 164 A1**
(43) Date of publication of application: **13.05.2015**
(21) Application number: 12880488.7
(22) Date of filing: 06.07.2012
(51) Int. Cl.: C02F 1/46, B01D 61/02, C02F 1/44

(54) **DEVICE FOR PRODUCING WATER FOR PREPARING DIALYSATE**

(71) Applicant: Nihon Trim Co., Ltd., Osaka 531-0076 (JP)
(72) Inventor: MORISAWA, Shinkatsu, Osaka-shi Osaka 531-0076 (JP)
(74) Representative: Lipscombe, Martin John
(86) International application number: PCT/JP2012/067310
(87) International publication number: WO 2014/006740

(57) **Abstract**

There is provided an apparatus (1) for producing water for preparation of a dialysis solution, including an electrolytic water generation device (2) that performs electrolysis using a solid polymer membrane (5), and a reverse osmosis membrane treatment device (3), the electrolytic water generation device being capable of reducing drained water as much as possible, and performing intense electrolysis to increase the amount of dissolved hydrogen without raising pH.

## Description

### TECHNICAL FIELD

The present invention relates to an apparatus for producing water for preparation of a dialysis solution.

### BACKGROUND ART

Dialysis is known as one effective treatment for patients with renal failure whose renal function is deteriorated such that he/she cannot urinate to adjust the amount of moisture and remove bodily toxic substances including bodily waste products, such as urea. Hemodialysis is a treatment that involves a continuous operation of drawing blood outside the body using a blood pump, bringing the blood in contact with a dialysis solution through a dialyzer to thereby remove bodily toxic substances and moisture by utilizing the phenomenon of diffusion based on the concentration gradient, and then returning the purified blood into the body.

In recent years, it is known that hemodialysis generates oxidative stress in dialysis patients. Since this is believed to be caused by active oxygen generated during dialysis, proposals have been made to reduce oxidative stress by eliminating active oxygen. Japanese Patent Laying-Open No. 2000-350989 (PTD 1), for example, discloses a method for producing a dialysis solution for hemodialysis. The method includes the steps of providing an electrolytic water generator having a cathode compartment including a cathode and an anode compartment including an anode, the cathode and anode compartments being separated from each other with a dividing membrane, supplying raw water containing at least sodium, potassium, magnesium, and calcium ions into each of the cathode and anode compartments, passing current between the anode and the cathode to electrolyze the raw water, and drawing out the water inside the cathode compartment. In the method, the water inside the cathode compartment has an actually measured value of oxidation-reduction potential in the range of -150 to 0 mV as measured using a platinum electrode, and a pH in the range of 8.0 to 9.5. The method is capable of eliminating active oxygen generated in the body.

In connection with this effect of reduced water, an apparatus for generating electrolyzed reduced water that is externally attached to an apparatus for generating reverse osmosis water, and an apparatus for readily generating reverse osmosis water made by integrating an apparatus for generating reduced water with a reverse osmotic membrane, have been developed.

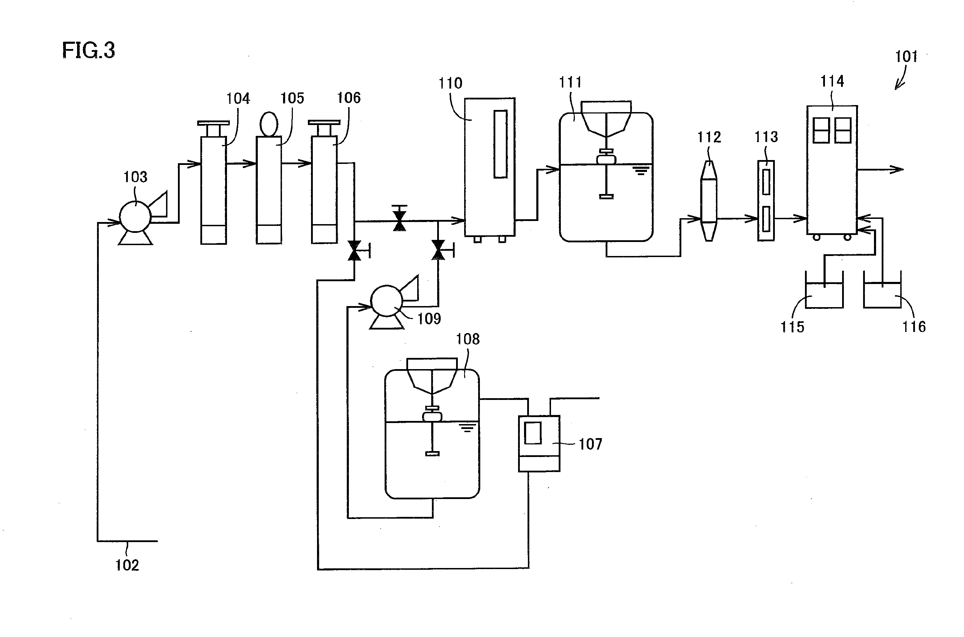

Here, Fig. 3 is a diagram showing a typical conventional water treatment system 101 for a dialysis solution. As shown in Fig. 3, for example, in conventional water treatment system 101 for a dialysis solution, raw water (municipal water) 102 is pressurized with a pressurizing pump 103, solids in raw water 102 are treated with a filter 104 having a pore size of 10 to 30 µm, hypochlorous acid contained in raw water 102 is removed with an activated carbon filtration device 105, and the hardness of raw water 102 is reduced with a softening device 106. Reduced water obtained by electrolyzing the raw water with an electrolytic water generation device 107 is then stored in a reduced water tank 108. The reduced water from reduced water tank 108 is pressurized with a pressurizing pump 109, and passed through a reverse osmotic membrane inside a reverse osmosis membrane treatment device 110. The reduced water that has passed through the reverse osmotic membrane is stored in an RO tank 111. The reduced water delivered from RO tank 111 is subjected to a sterilization treatment with an UV sterilization lamp 112, passed through a microfilter 113, and supplied to a dialysis solution supply device 114. Dialysis solution supply device 114 mixes the supplied reduced water that has been subjected to the reverse osmosis membrane treatment with a dialysis A raw solution 115 and a dialysis B raw solution 116, and supplies the mixture to each patient monitor device (not shown) as a dialysis solution. A patient's blood is then purified through a dialyzer attached to the patient monitor device.

Moreover, Japanese Patent Laying-Open No. 2010-63629 (PTD 2), for example, discloses a dialysis apparatus utilizing reduced water. The dialysis apparatus includes gas-liquid mixing means for mixing water with hydrogen and causing dissolution of the hydrogen in the water by pressurization, microbubble generating means for generating microbubbles in the water containing the dissolved hydrogen obtained by the gas-liquid mixing means, and dialysis solution supplying means for supplying the water containing the microbubbles obtained by the microbubble generating means as a dialysis solution. With the dialysis apparatus as described in PTD 2, a dialysis solution capable of eliminating active oxygen in the blood of a dialysis patient can be supplied. Moreover, since electrolysis is not performed, anode water that has conventionally been drained without being used is not generated, thus leading to a reduced amount of drained water.

### CITATION LIST

### PATENT DOCUMENT

PTD 1: Japanese Patent Laying-Open No. 2000-350989
PTD 2: Japanese Patent Laying-Open No. 2010-63629

### SUMMARY OF INVENTION

### TECHNICAL PROBLEM

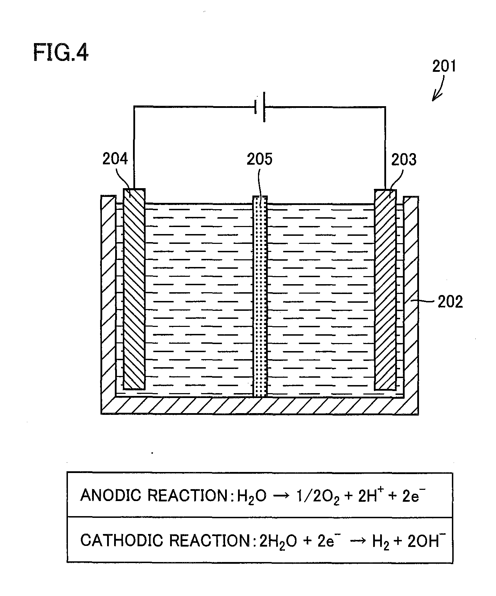

Fig. 4 is a schematic diagram showing an electrolytic cell 202 used in a typical conventional electrolytic water generation device 201. Generally, as reduced water, water is used which is obtained at a cathode 204-side by electrolysis within electrolytic cell 202 in which a dividing membrane 205 is placed between an anode 203 and a cathode 204. In electrolysis, the following reactions occur at the anode 203-side and the cathode 204-side:
Anode Side: H₂O → 1/2O₂ + 2H⁺ + 2e⁻
Cathode Side: 2H₂O + 2e⁻ → H₂ + 2OH⁻

By the electrolysis shown above, electrolyzed acid water is generated at the anode 203-side of electrolytic cell 202 and drained. This draining of the electrolyzed acid water not only increases the water charge, but can also be regarded as wasting natural resources. Reducing the amount of drained water, therefore, has a significant advantage. In the electrolytic cell of a usual electrolytic water generation device, however, elimination of drained electrolyzed acid water is not possible based on its principles. Moreover, if the amount of drained electrolyzed acid water is reduced, hypochlorous acid and the like generated at the anode side are mixed into reduced water, and therefore, the amount of drained water that can be reduced has been limited.

Furthermore, in the typical conventional electrolytic water generation device 201, the anode 203-side becomes acidic due to the generation of H⁺ ions, and the cathode 204-side becomes alkaline due to the generation of OH⁻ ions. Since the amount of dissolved hydrogen is proportional to the intensity of electrolysis, increasing dissolved hydrogen increases alkalinity at the cathode 204-side. It is, however, considered to be undesirable to pass water having a pH of 10 or higher as water to be passed through a reverse osmotic membrane for application to a dialysis apparatus, and the amount of dissolved hydrogen has also been restricted by the pH limit.

The present invention was made to solve the above-described problem, and an object of the invention is to provide an apparatus for producing water for preparation of a dialysis solution, including an electrolytic water generation device that is capable of reducing drained water as much as possible, and performing intense electrolysis to increase the amount of dissolved hydrogen without raising pH.

### SOLUTION TO PROBLEM

An apparatus for producing water for preparation of a dialysis solution according to the present invention includes an electrolytic water generation device that performs electrolysis using a solid polymer membrane, and a reverse osmosis membrane treatment device.

In the apparatus for producing water for preparation of a dialysis solution according to the present invention, water that has passed through the reverse osmosis membrane treatment device may be electrolyzed by the electrolytic water generation device, or water generated by the electrolytic water generation device may be passed through the reverse osmosis membrane treatment device.

### ADVANTAGEOUS EFFECTS OF INVENTION

According to the present invention, since the electrolytic water generation device using a solid polymer membrane is applied, it is unnecessary to drain water formed at the anode side after electrolysis, and an amount of water that has decreased by electrolysis may only be supplied. Moreover, in the electrolytic water generation device using a solid polymer membrane, since the pH does not change before and after electrolysis, water that has been subjected to intense electrolysis can be supplied to the reverse osmosis membrane treatment device. Since the pH does not change even if a high voltage is applied to increase the amount of dissolved hydrogen, the water after being subjected to such electrolysis can be subjected to a reverse osmosis membrane treatment. Furthermore, since electrolytic water generation device 2 using a solid polymer membrane 5 can also electrolyze pure water, water after being subjected to the reverse osmosis membrane treatment in a reverse osmosis membrane treatment device 3 may be electrolyzed by electrolytic water generation device 2. This obviates the need to provide a separate tank for storing the water after electrolysis in electrolytic water generation device 2 or a pump therefor, in addition to a tank (RO tank) 21 for storing water after the reverse osmosis membrane treatment. This leads to reductions in size and cost. Furthermore, deposition of minerals does not occur in electrolytic water generation device 2 using solid polymer membrane 5. This also achieves an effect of preventing electrolysis from being hindered by deposition of minerals.

### BRIEF DESCRIPTION OF DRAWINGS

Fig. 1 is a schematic diagram showing an apparatus 1 for producing water for preparation of a dialysis solution in one preferred embodiment of the present invention;
Fig. 2 is a schematic diagram showing an electrolytic cell 4 in electrolytic water generation device 2 in apparatus 1 for producing water for preparation of a dialysis solution according to the present invention;
Fig. 3 is a diagram showing typical conventional water treatment system 101 for a dialysis solution; and
Fig. 4 is a schematic diagram showing electrolytic cell 202 used in typical conventional electrolytic water generation device 201.

### DESCRIPTION OF EMBODIMENTS

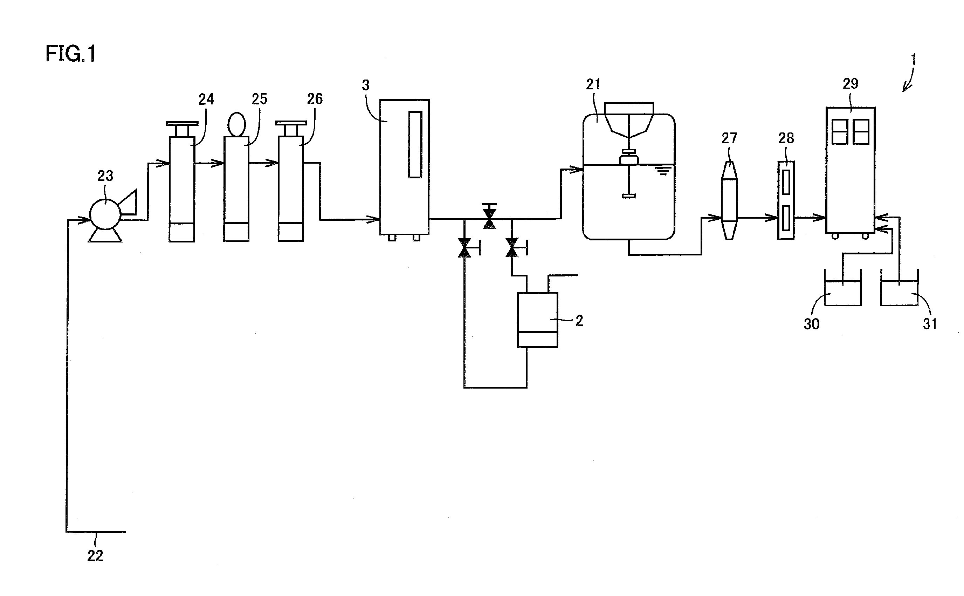

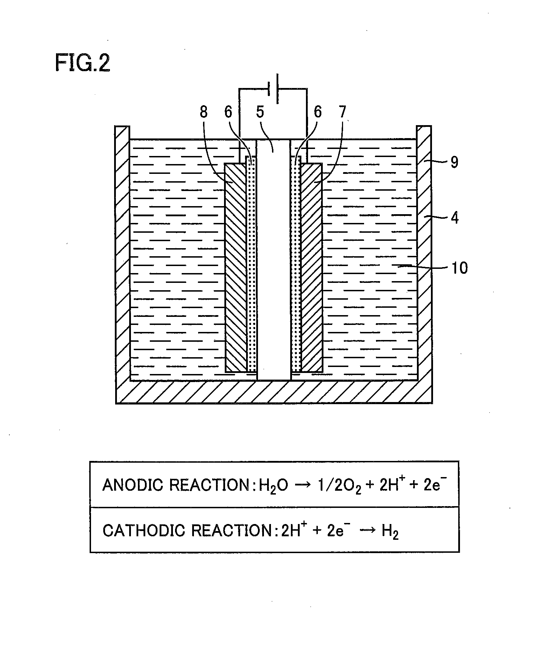

Fig. 1 is a schematic diagram showing apparatus 1 for producing water for preparation of a dialysis solution in one preferred embodiment of the present invention, and Fig. 2 is a schematic diagram showing electrolytic cell 4 in electrolytic water generation device 2 in apparatus 1 for producing water for preparation of a dialysis solution according to the present invention. As shown in Fig. 2, apparatus 1 for producing water for preparation of a dialysis solution according to the present invention includes electrolytic water generation device 2 that performs electrolysis using solid polymer membrane 5, and reverse osmosis membrane treatment device 3.

As solid polymer membrane 5 used in electrolytic water generation device 2 in the present invention, a suitable solid polymer membrane that is conventionally used in the field of fuel cells can be used without being particularly limited. For example, solid polymer membrane 5 formed of a resin material having an ion-exchange function, such as fluorine-based ion-exchange resin, can be suitably used. Specifically, a commercially available product such as Nafion (from Du Pont), Flemion (from Asahi Glass Co., Ltd.), Aciplex (from Asahi Glass Co., Ltd.), or the like can be suitably used as solid polymer membrane 5 in the present invention.

As shown in Fig. 2, in electrolytic water generation device 2 used in the present invention, a structure in which an anode 7 and a cathode 8 electrically connected to each other are placed with a metal layer 6 formed on opposing surfaces of solid polymer membrane 5 being present therebetween, is immersed in electrolytic cell 9 containing water 10 to be electrolyzed.

Examples of materials of anode 7 include those obtained by coating conductive substrates such as titanium, tantalum, niobium, nickel, zirconium, SUS, and the like, with platinum and/or iridium alone or in combination.

Examples of materials of cathode 8 also include those obtained by coating conductive substrates such as titanium, tantalum, niobium, nickel, zirconium, SUS, and the like, with platinum and/or iridium alone or in combination.

Examples of metal materials forming metal layer 6 present between anode 7 and solid polymer membrane 5 and between cathode 8 and solid polymer membrane 5 include platinum (Pt) and/or iridium (Ir) alone or in combination. The thickness of metal layer 6 is not particularly limited.

Electrolytic cell 9 is not particularly limited, and a suitable electrolytic cell used for electrolysis in the pertinent field may be used as electrolytic cell 9.

In electrolysis in electrolytic water generation device 2 using solid polymer membrane 5 as described above, the following reactions occur at an anode 7 side and at a cathode 8 side:
Anode Side: H₂O → 1/2O₂ + 2H⁺ + 2e⁻
Cathode Side: 2H⁺ + 2e⁻ → H²

In electrolytic water generation device 2 using solid polymer membrane 5 as described above, it is unnecessary to drain water formed at the anode 7 side after electrolysis, and an amount of water that has decreased by electrolysis may only be supplied. Hence, although it is necessary to remove oxygen gas generated, the amount of drained water can be reduced to almost zero, as compared to conventional electrolytic water generation devices.

Furthermore, in electrolytic water generation device 2 using solid polymer membrane 5, the pH does not change before and after electrolysis. Since the pH does not change even if intense electrolysis is performed by applying a high voltage in order to increase the amount of dissolved hydrogen, even when the water after being subjected to such electrolysis is supplied to the reverse osmosis membrane treatment device, it can be subjected to a reverse osmosis membrane treatment without damage to the reverse osmotic membrane.

Alternatively, since electrolytic water generation device 2 using solid polymer membrane 5 can also electrolyze pure water, the water after being subjected to the reverse osmosis membrane treatment in reverse osmosis membrane treatment device 3 may be electrolyzed by electrolytic water generation device 2. In this way, according to the present invention, water that has passed through reverse osmosis membrane treatment device 3 may be electrolyzed by electrolytic water generation device 2, or water generated by electrolytic water generation device 2 may be passed through reverse osmosis treatment device 3. Fig. 1 shows the case where the water that has passed through reverse osmosis membrane treatment device 3 is electrolyzed in electrolytic water generation device 2. This obviates the need to provide a separate tank for storing the water after electrolysis in electrolytic water generation device 2 or a pump therefor, in addition to a tank (RO tank) 21 for storing water after the reverse osmosis membrane treatment. This leads to reductions in size and cost.

Furthermore, in conventionally used electrolytic water generation devices, deposition of minerals such as calcium (Ca) and the like on a cathode surface hindered electrolysis. In contrast, in electrolytic water generation device 2 using solid polymer membrane 5 as in the present invention, deposition of minerals does not occur, so that electrolysis can be prevented from being hindered by such deposition of minerals.

As reverse osmosis membrane treatment device 3 in apparatus 1 for producing water for preparation of a dialysis solution according to the present invention, a conventionally known suitable reverse osmosis (RO) device can be used without being particularly limited, and specifically, HM500CX (from Japan Water Systems Corporation), for example, is preferable. As used herein, the reverse osmosis membrane treatment refers to the following treatment: when there are solutions with different concentrations with a semi-permeable membrane as a boundary between them, pressure is applied toward a solution with a higher concentration to thereby obtain water that has permeated through a solution with a lower concentration, as opposed to osmosis that is a phenomenon in which water moves from the solution with a lower concentration toward the solution with a higher concentration. By this reverse osmosis membrane treatment, impurities such as trace metals can further be removed from the raw water obtained by the series of treatments described above, and the resulting water can thus meet the water quality standards defined in ISO 13959 described below.

Apparatus 1 for producing water for preparation of a dialysis solution according to the present invention is not particularly limited in structure, except for electrolytic water generation device 2 using solid polymer membrane 5 and reverse osmosis membrane treatment device 3 described above, and can have a structure similar to those of conventional apparatuses for producing water for preparation of dialysis solutions. For example, in exemplary apparatus 1 for producing water for preparation of a dialysis solution shown in Fig. 1, raw water (municipal water) 22 is pressurized with a pressurizing pump 23, treated with a filter 24, and then sequentially subjected to treatments with an activated carbon filtration device 25 and a softening device 26.

In apparatus 1 for producing water for preparation of a dialysis solution according to the present invention, tap water, well water, or ground water can be used as raw water 22. As filter 24 for filtering raw water 22 to remove coarse foreign substances such as iron rust (precipitated from a supply pipe), sand, and the like contained in raw water 22, a filter with a pore size of 10 to 30 µm can be suitably used, and specifically, a 25 µm filter (from Japan Water Systems Corporation), a 10 µm filter (from Japan Water Systems Corporation), or the like is preferable.

Activated carbon filtration device 25 is for subjecting the raw water to a treatment to remove residual chlorine, chloramine, organic substances and the like contained in the raw water by a physical adsorption effect, using activated carbon, which is a porous adsorptive material. As activated carbon filtration device 25, a conventionally known suitable activated carbon treatment device can be used without being particularly limited, and specifically, fibrous activated carbon MOF250C2 (from Futamura Chemical Co., Ltd.), for example, is preferable.

Softening device 26 is for subjecting the raw water, which is hard water containing dissolved solids (calcium ion, magnesium ion, and the like) as hard water components, to a treatment to turn the raw water into soft water by removing the hard water components through a replacement reaction using ion exchange. As softening device 26, a conventionally known suitable softening device can be used without being particularly limited, and specifically, MARK-915U (from Japan Water Systems Corporation), for example, is preferable.

Water subjected to the reverse osmosis membrane treatment that has passed through reverse osmosis membrane treatment device 3 is thereafter supplied to electrolytic water generation device 2 for electrolysis, and then stored in an RO tank 21. Reduced water delivered from RO tank 21 is subjected to a sterilization treatment with an UV sterilization lamp 27, passed through a microfilter 28, and supplied to a dialysis solution supply device 29. Dialysis solution supply device 29 mixes the supplied reduced water that has been subjected to the reverse osmosis membrane treatment with a dialysis A raw solution 30 and a dialysis B raw solution 31, and supplies the mixture to each patient monitor device (not shown) as a dialysis solution. A patient's blood is then purified through a dialyzer attached to the patient monitor device.

### REFERENCE SIGNS LIST

1: apparatus for producing water for preparation of a dialysis solution; 2: electrolytic water generation device; 3: reverse osmosis membrane treatment device; 4: electrolytic cell; 5: solid polymer membrane; 6: metal layer; 7: anode; 8: cathode; 9: electrolytic cell; 10: water; 21: RO tank; 22: raw water; 23: pressurizing pump; 24: filter; 25: activated carbon filtration device; 26: softening device; 27: UV sterilization lamp; 28: microfilter; 29: dialysis solution supply device; 30: dialysis A raw solution; 31: dialysis B raw solution.

## Claims

1. An apparatus (1) for producing water for preparation of a dialysis solution, comprising:
an electrolytic water generation device (2) that performs electrolysis using a solid polymer membrane (5); and
a reverse osmosis membrane treatment device (3).

2. The apparatus (1) according to claim 1, wherein
water that has passed through the reverse osmosis membrane treatment device (3) is electrolyzed by the electrolytic water generation device (2).

3. The apparatus (1) according to claim 1, wherein
water generated by the electrolytic water generation device (2) is passed through the reverse osmosis membrane treatment device (3).
